# EUROPEAN PATENT APPLICATION

(11) **EP 4 649 893 A1**
(43) Date of publication of application: **19.11.2025**
(21) Application number: 24305737.9
(22) Date of filing: 13.05.2024
(51) Int. Cl.: A61B 8/06, A61B 8/08

(54) **2D PISA WITH 3D DATA**

(71) Applicant: Koninklijke Philips N.V., 5656 AG Eindhoven (NL)
(72) Inventor: SU, Jimmy, 5656 Eindhoven (NL); BONNEFOUS, Odile, 5656 Eindhoven (NL)
(74) Representative: Philips Intellectual Property & Standards

(57) **Abstract**

Proposed concepts thus aim to provide schemes, solutions, concept, designs, methods and systems pertaining to automatically determining an iso-velocity surface for use in 2D PISA measurements. In particular, embodiments aim to provide a method for automatically determining an iso-velocity surface for use in 2D PISA measurements by using a blood flow model of the regurgitant flow in order to automatically determine an area and location of a flow convergence zone in the blood flow model. Using the determined area and location of the flow convergence zone, an iso-velocity surface can be determined corresponding to a 2D PISA hemisphere.

## Description

### FIELD OF THE INVENTION

This invention relates to the field of 2D PISA measurements.

### BACKGROUND OF THE INVENTION

Echocardiography plays a pivotal role in clinical practice in defining the mechanism and severity of cardiac valve regurgitation, such as mitral regurgitation (MR), requiring the integration of multiple parameters for categorization of mitral regurgitation grade. In MR, the mitral valve does not close properly and therefore allows backflow of blood from the left ventricle into the left atrium when the left ventricle contracts (systole).

Many of the quantitative approaches to MR rely upon the principles of flow convergence, which are embedded within the 2D proximal iso-velocity surface area (PISA) method to estimate regurgitant orifice area (EROA) size and regurgitant volume (RVol). The limitations of the PISA method are largely due to attendant suppositions regarding the geometry of the orifice and flow convergence, along with the assumption of temporal constancy of flow during systole.

2D PISA, by nature, is very subjective and user-specific. The user acquires a 2D color Doppler image, making sure to capture the appropriate diameter of the regurgitant orifice at the peak of the regurgitation. The user then adjusts the color baseline to find a semicircle shape of the PISA hemisphere. The radius of that hemisphere is then manually measured from border of that semicircle to the region of narrowest color (which is assumed to be the regurgitant orifice). The radius is used to find the PISA area, which, when multiplied by the aliasing speed, gives the regurgitant flow in the PISA. Dividing that flow by the maximum velocity of mitral regurgitation gives the area of the orifice (EROA). The EROA multiplied by the Velocity Time Integral (VTI) then provides the user with the RVol, as determined by PISA.

As can be seen, there are many assumptions being made here in 2D PISA, and the calculations themselves can be variable due to user selection. One comment that has been oft repeated by physicians, even those who use PISA regularly, is that any result can be obtained from PISA just by adjusting the manual measurements ever so slightly, which reduces the reproducibility of the technique. Furthermore, even with the most careful radius measurements, the radius value is squared, which compounds any errors or variations in the measurement.

### SUMMARY OF THE INVENTION

The invention is defined by the claims.

According to examples in accordance with an aspect of the invention, there is provided a computer-implemented method for automatically determining an iso-velocity surface for use in 2D proximal iso-velocity surface (PISA) measurements.

The method comprises: obtaining a blood flow model of regurgitant flow, wherein the blood flow model comprises a model of an orifice and blood flow velocity values through and around the orifice; determining a surface area and location of a flow convergence zone in the blood flow model based on blood flow velocity values of the blood flow model; and determining an iso-velocity surface of the blood flow model based on the determined surface area and location of the flow convergence zone.

Proposed concepts thus aim to provide schemes, solutions, concepts, designs, methods and systems pertaining to automatically determining an iso-velocity surface for use in 2D PISA measurements. In particular, embodiments aim to provide a method for automatically determining an iso-velocity surface for use in 2D PISA measurements by using a blood flow model of the regurgitant flow in order to automatically determine an area and location of a flow convergence zone in the blood flow model. Using the determined area and location of the flow convergence zone, an iso-velocity surface can be determined corresponding to a 2D PISA hemisphere.

In other words, it is proposed that, by using a blood flow model of the regurgitant flow (which is generated using 3D Doppler data representative of regurgitant flow associated with the orifice in the cardiac valve), a 2D PISA hemisphere can essentially be determined automatically, without any need for user intervention, thus minimizing the variability of the PISA method while still providing clinicians with an expected and familiar component of the PISA method. The automatically determined iso-velocity surface (PISA hemisphere) can then be used traditionally, e.g., to determine an area of the orifice and the regurgitant flow volume.

By using 3D data, i.e., via the blood flow model (and optionally, additionally with 3D Doppler data directly), enough information is obtained to be able to accurately determine an iso-velocity surface (PISA hemisphere) automatically. There are, of course, many ways that the obtained blood flow model (and optionally, separate 3D Doppler data) could be analyzed and used, but by using it to essentially determine a 2D PISA hemisphere, a clinician is provided with a familiar output which they will feel comfortable with and can intuitively get a sense of whether it is reliable or not, for example.

For example, by automating the process of determining an iso-velocity surface (corresponding to a 2D PISA hemisphere), the variability in 2D PISA can be reduced, thus giving users more confidence in 2D PISA results, aligning with MR severity guidelines.

In yet other words, the present concept facilitates automatic 2D Proximal Iso-velocity Surface (PISA) calculation using 3D echocardiography data, for example, in order to assist clinicians to consistently and repeatably perform 2D PISA measurements on mitral (or tricuspid or aortic) valve regurgitation echo data. The method essentially leverages 3D data to reduce inter- and intra- user variability.

Ultimately, an improved method for dynamically adjusting Doppler data frames of regurgitant flow in a cardiac valve is provided.

In some embodiments, the method may further comprise: obtaining 3D Doppler data representative of the regurgitant flow associated with the orifice in the cardiac valve; and determining a size and location of the orifice in the blood flow model based on the 3D Doppler data; wherein determining the iso-velocity surface of the blood flow model is further based on the determined size and location of the orifice. Using additional 3D Doppler data directly allows the iso-velocity surface of the blood flow model to be additionally based on a determined size and location of the orifice. This facilitates the orifice essentially acting as a central anchor point of the iso-velocity surface, such that the flow convergence zone can essentially be converted into the iso-velocity surface (for example, in the form of a hemisphere), with the orifice being used as the center-point of said iso-velocity surface (e.g., hemisphere). For instance, the average radius from the center or edges of the orifice could be used as the constant radius for a hemispherical iso-velocity surface centered on the orifice, thus improving the results of the method.

Therefore, some embodiments of the present invention aim to provide a method for automatically determining an iso-velocity surface for use in 2D PISA measurements by using both 3D Doppler data and a blood flow model of the regurgitant flow in order to automatically determine an area and location of both the orifice and the flow convergence zone in the blood flow model. Using the determined areas and locations of the orifice and the flow convergence zone respectively, an iso-velocity surface can be determined corresponding to a 2D PISA hemisphere.

In some embodiments, the iso-velocity surface may comprise a spherical surface. This may facilitate the determined iso-velocity surface being more compatible with traditional 2D PISA measurements, allowing the iso-velocity surface to be used as a traditional 2D PISA hemisphere. This may also simplify the calculation and computations needed to determine the iso-velocity surface, thereby improving the efficiency of the method.

In some embodiments, the method may further comprise obtaining a velocity time integral of the regurgitant flow and determining a regurgitant flow volume based on the iso-velocity surface of the blood flow model and the velocity time integral. This may facilitate a more comprehensive automatic assessment of the regurgitant flow by quantifying the total volume of the regurgitation flow, which is clinically relevant for patient diagnosis and treatment planning.

In some embodiments, the method may further comprise obtaining 2D continuous wave Doppler data representative of the regurgitant flow, and determining the velocity time integral based on the obtained 2D continuous wave Doppler data. This may allow the velocity time integral to be directly automatically determined in the method rather than obtained from an external source, and thus allows the method to be more self-contained.

In some embodiments, the size of the orifice may be determined as a diameter of a circle and the surface area of the flow convergence zone may be determined as spherical. Standardizing the shape of the orifice and flow convergence zone to geometrically simple forms may facilitate more efficient and consistent determination of the iso-velocity surface, as well as making the automated computation more comparable with current standard clinical methods.

In some embodiments, the 3D Doppler data may comprise at least two 3D Doppler data frames from a temporal sequence, and wherein the blood flow model may comprise at least two blood flow model frames from a temporal sequence. This may provide a dynamic view of the regurgitant flow over time, which can capture variations within the cardiac cycle and potentially lead to more accurate assessments.

In some embodiments, the size and location of the orifice may be based on the 3D Doppler data frame comprising the largest regurgitant jet or the largest orifice area respectively. Focusing on the frame with the greatest regurgitation may ensure that the determined iso-velocity surface accurately reflects the extent of the regurgitation.

In some embodiments, the surface area and location of the flow convergence zone may be based on the blood flow velocity values from the blood flow model frame comprising the largest regurgitant flow or the largest orifice area respectively. Focusing on the frame with the greatest regurgitation may ensure that the determined iso-velocity surface accurately reflects the extent of the regurgitation.

In some embodiments, a size and location of the orifice may be determined for each Doppler data frame, a surface area and location of the flow convergence zone may be determined for each blood flow model frame, and an iso-velocity surface may be determined for each pair of corresponding frames. This allows for a frame-by-frame analysis, which may provide a detailed temporal representation of the regurgitant flow, facilitating a more nuanced understanding of the condition.

In some embodiments, the method may further comprise detecting cardiac valve landmarks in the blood flow model of the regurgitant flow, and wherein determining the size and location of the orifice may be further based on the detected cardiac valve landmarks and/or wherein determining the surface area and location of the flow convergence zone may be further based on the detected cardiac valve landmarks. Incorporating anatomical landmarks may improve the accuracy of the model by ensuring that the measurements are anatomically consistent.

In some embodiments, the method may further comprise generating a display control signal, the display control signal describing the determined iso-velocity surface. This may facilitate the visualization of the iso-velocity surface, which can aid clinicians in interpreting the data and making informed decisions regarding patient care and/or checking that the automatic determination of the 2D PISA hemisphere agrees with their expectations.

According to another aspect of the invention, there is provided a computer program comprising code means for implementing the method of any herein disclosed method when said program is run on a processing system.

According to another aspect of the invention, there is provided a system for automatically determining an iso-velocity surface for use in 2D PISA measurements. The system comprising an input interface configured to: obtain a blood flow model of regurgitant flow, wherein the blood flow model comprises a model of an orifice in a cardiac valve and blood flow velocity values through and around the orifice; and a processing unit configured to: determine a surface area and location of a flow convergence zone in the blood flow model based on blood flow velocity values of the blood flow model; and determine an iso-velocity surface of the blood flow model based on the determined surface area and location of the flow convergence zone.

In some embodiments, the input interface may be further configured to: obtain 3D Doppler data representative of regurgitant flow associated with the orifice in the cardiac valve; and wherein the processing unit may be further configured to: determine a size and location of the orifice in the blood flow model based on the 3D Doppler data; and wherein determining the iso-velocity surface of the blood flow model may be further based on the determined size and location of the orifice.

In some embodiments, the iso-velocity surface may comprise a spherical surface.

In some embodiments, the processing unit may be further configured to obtain a velocity time integral of the regurgitant flow and determine a regurgitant flow volume based on the iso-velocity surface of the blood flow model and the velocity time integral.

Thus, there may be proposed concepts for automatically determining an iso-velocity surface for use in 2D PISA measurements, and this may be done based on 3D Doppler data and a blood flow model of the regurgitant flow.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a computer-implemented method for automatically determining an iso-velocity surface for use in 2D PISA measurements according to a proposed embodiment;
Fig. 2 is a diagram showing regurgitant flow in a cardiac valve;
Fig. 3 is a flow diagram of a computer-implemented method for automatically determining an iso-velocity surface for use in 2D PISA measurements according to a proposed embodiment;
Fig. 4 is a flow diagram of a computer-implemented method for automatically determining an iso-velocity surface for use in 2D PISA measurements according to a proposed embodiment;
Fig. 5 is a flow diagram of a computer-implemented method for automatically determining an iso-velocity surface for use in 2D PISA measurements according to a proposed embodiment;
Fig. 6 is a simplified block diagram of a system for automatically determining an iso-velocity surface for use in 2D PISA measurements according to a proposed embodiment; and
Fig. 7 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to automatically determining an iso-velocity surface for use in 2D PISA measurements. According to proposed concepts, a number of possible solutions may be implemented separately or jointly. That is, although these possible solutions may be described below separately, two or more of these possible solutions may be implemented in one combination or another.

Embodiments of the invention aim to provide a method for automatically determining an iso-velocity surface for use in 2D PISA measurements. This can be achieved by using a blood flow model of the regurgitant flow in order to automatically determine an area and location of a flow convergence zone in the blood flow model. Using the determined area and location of the flow convergence zone, an iso-velocity surface can be determined corresponding to a 2D PISA hemisphere.

In other words, it is proposed that, by using a blood flow model of the regurgitant flow (which is generated using 3D Doppler data representative of regurgitant flow associated with the orifice in the cardiac valve), a 2D PISA hemisphere can essentially be determined automatically, without any need for user intervention, thus minimizing the variability of the PISA method while still providing clinicians with an expected and familiar component of the PISA method. The automatically determined iso-velocity surface (PISA hemisphere) can then be used traditionally, e.g., to determine an area of the orifice and the regurgitant flow volume.

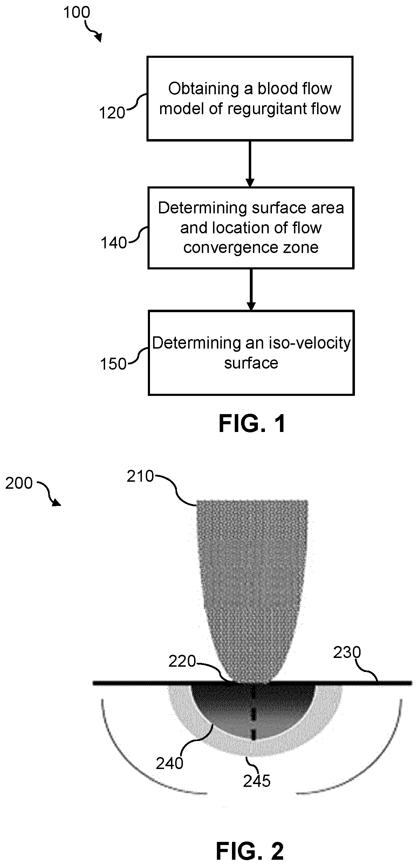

Referring now to Fig. 1, there is depicted a simplified flow diagram of a method 100 for automatically determining an iso-velocity surface for use in 2D PISA measurements according to a proposed embodiment.

In step 120, a blood flow model of regurgitant flow is obtained, wherein the blood flow model comprises a model of an orifice in a cardiac valve (such as a mitral, tricuspid or aortic valve) and blood flow velocity values through and around the orifice. For example, the blood flow model can comprise a 3D vector model of the orifice and blood flow through and around the orifice. For example, the blood flow model can be obtained from an external source, such as a memory storage. In some embodiments, the blood flow model can be obtained by generating the blood flow model according to any suitable method, as would be understood by the skilled person, such as, for example, according to the concepts described in WO 2023/020920 A1.

In step 140, a surface area and location of a flow convergence zone in the blood flow model is determined based on blood flow velocity values of the blood flow model. The flow convergence zone refers to the region in which blood flow converges towards a regurgitant orifice within the heart, forming a hemispheric shape due to the continuity principle. The flow convergence zone is computed as a 3D shape (e.g., a hemisphere) and therefore the surface area of the flow convergence zone is the surface area of this 3D shape.

In step 150, an iso-velocity surface of the blood flow model is determined based on the determined surface area and location of the flow convergence zone. The flow convergence zone is the same as the iso-velocity surface in 2D, in that the iso-velocity surface is assumed to be a spherical hemisphere, through making a 2D measurement of radius. In traditional 2D PISA, flow convergence is detected by doing a color baseline shift to find the border of the red-blue transition (for example). Instead of doing a color baseline shift, the current invention uses the blood flow model to detect the flow convergence zone in 3D automatically, which is then estimated into a spherical hemisphere as the iso-velocity surface in order to be compatible with 2D PISA measurements. In other words, the flow convergence zone is essentially converted into the iso-velocity surface (e.g., via approximating it as a spherical hemisphere).

Referring now to Fig. 2, there is depicted a diagram 200 showing regurgitant flow in a cardiac valve 230. For example, an orifice 220 is provided in a cardiac valve 230 (such as a mitral valve). A liquid, such as blood or any other liquid, may thus pass from one side of the cardiac valve 230 to the other side through the orifice 220. Iso-velocity curves (i.e., potential iso-velocity surfaces), 240 and 245, are positioned around the orifice 220. For example, positioned around the orifice 220 is a first iso-velocity curve 240 and a second iso-velocity curve 245. The iso-velocity curves, 240 and 245, can thus represent the velocity of the fluid as it approaches the orifice 220 before passing through it, resulting in a fluid jet 210.

For example, as the fluid approaches the orifice 220, it may accelerate, reaching a maximum velocity as it passes through the orifice 220. As a result, the fluid positioned at the first iso-velocity curve 240 may be of a constant velocity at all points along the curve 240. In addition, although the curves, 240 and 245, are shown as two-dimensional, the iso-velocity curves can be represented as three-dimensional hemispheric shaped shells representing all points within the fluid surrounding the orifice 220 of the same velocity.

The iso-velocity curves, 240 and 245, are observed upstream of the orifice 220. Fluid in this particular example may thus flow from below the cardiac valve 230 to above the cardiac valve 230.

For example, in clinical practice today, regurgitant flow quantification with ultrasound typically relies on the 2D Proximal Iso-velocity Surface Area (PISA) principle. The 2D PISA method employs a proximal flow convergence zone to measure the volume of regurgitation.

The principle underlying this method is straightforward and based on simple fluid dynamics. The flow convergence zone corresponds to regurgitant flow. Blood flow velocity increases as it approaches the regurgitant orifice 220. In the proximal flow convergence region, the blood flows through successive iso-velocity surfaces, 245 and 240, wherein iso-velocity means that the velocity on the surface of each individual iso-velocity surface is equal.

The PISA method employs the proximal flow convergence zone to measure the volume of regurgitation. It assumes that the flow convergence zone corresponds to regurgitant flow. Blood flow velocity increases as it approaches the regurgitant orifice 220. As described above, the proximal flow convergence zone can thus be described as hemispheric shells, 240 and 245, in which the velocity on the surface of each of the shells is equal.

The inventors thus propose to significantly modify the PISA method by using 3D Doppler data and a blood flow model of the regurgitant flow in order to automatically determine an iso-velocity surface (e.g., automatically determine a center and a radius of a spherical surface). The automatically determined radius, for example, can then be used as normal in the 2D PISA method to determine a regurgitant flow volume which is less reliant on a specific user's judgement of where to place the iso-velocity surface.

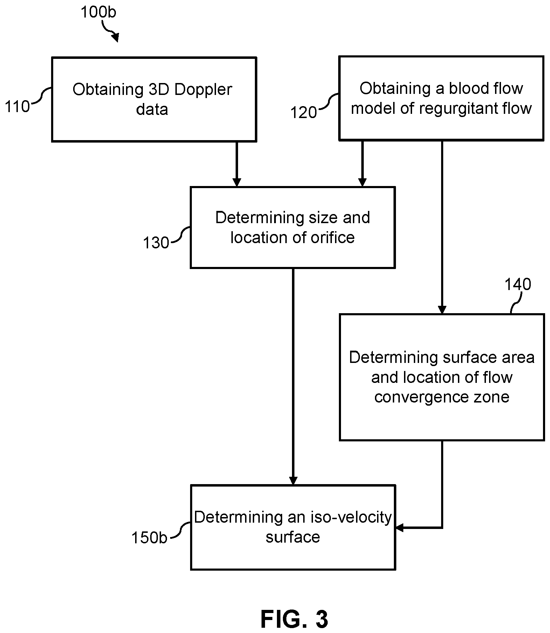

Referring now to Fig. 3, there is depicted a simplified flow diagram of a method 100b for automatically determining an iso-velocity surface for use in 2D PISA measurements according to a proposed embodiment. Steps 120 and 140 are substantially the same as have bene described in relation to method 100 of Fig. 1.

Step 110 comprises obtaining 3D Doppler data representative of the regurgitant flow associated with the orifice in the cardiac valve. For example, the 3D Doppler data can comprise 3D color Doppler volume data of a cardiac valve throughout at least part of a cardiac cycle. For example, the 3D Doppler data can be obtained from an external source, such as a memory storage. In some embodiments, step 110 may comprise actually capturing the 3D Doppler data representative of regurgitant flow associated with an orifice in a cardiac valve. For example, using 3D data can be used to solve any issues with foreshortening or off-axis images of the regurgitation, as is often the case with 2D PISA.

Of course, the skilled person would understand that steps 110 and 120 can be performed in any order.

In step 130, a size and location of the orifice in the blood flow model is determined based on the 3D Doppler data. A size/area of the orifice (also referred to as the hole of regurgitation) is treated as a planar surface. In some embodiments, the orifice can be modelled as many small holes positioned next to each other in the shape of the orifice - in this case, the size/area of the orifice can be understood as the sum of the sizes/areas of all the small holes put together.

In this embodiment, the size of the orifice is determined as a diameter of a circle. Standardizing the shape of the orifice to a geometrically simple form facilitates more efficient and consistent determination of the iso-velocity surface. Of course, the skilled person would understand that in other embodiments, the size/area of the orifice can be determined in any suitable way.

In some embodiments, determining the flow convergence zone in step 140 can further be based on the 3D Doppler data.

In this embodiment, the surface area of the orifice is determined as spherical (or hemispherical). Standardizing the shape of the orifice to a geometrically simple form facilitates more efficient and consistent determination of the iso-velocity surface. Of course, the skilled person would understand that in other embodiments, the surface area of the flow convergence zone can be determined in any suitable way.

In step 150b, an iso-velocity surface of the blood flow model is determined based on the determined surface area and location of the flow convergence zone and the determined size and location of the orifice. As described above, the flow convergence zone is the same as the iso-velocity surface in 2D, in that the iso-velocity surface is assumed to be a spherical hemisphere, through making a 2D measurement of radius. By further considering 3D Doppler data, the determined orifice can essentially act as a central anchor point of the iso-velocity surface, such that the flow convergence zone can essentially be converted into the iso-velocity surface (for example, in the form of a hemisphere), with the orifice being used as the center-point of said iso-velocity surface (e.g., hemisphere). For instance, the average radius from the center or edges of the orifice could be used as the constant radius for a hemispherical iso-velocity surface centered on the orifice, thus improving the results of the method (e.g., resulting in an improved iso-velocity surface for the purposes of 2D PISA). Of course, the skilled person would understand the many ways the size and location of the orifice could be used to aid the determining of the iso-velocity surface.

In this embodiment, the iso-velocity surface comprises a spherical surface. This facilitates the determined iso-velocity surface being compatible with traditional 2D PISA measurements, allowing the iso-velocity surface to be used as a traditional 2D PISA hemisphere. This also simplifies the calculations and computations needed to determine the iso-velocity surface, thereby improving the efficiency of the method. It is noted that spherical can also include hemispherical. Of course, in other embodiments, the iso-velocity surface can comprise a surface of any suitable shape.

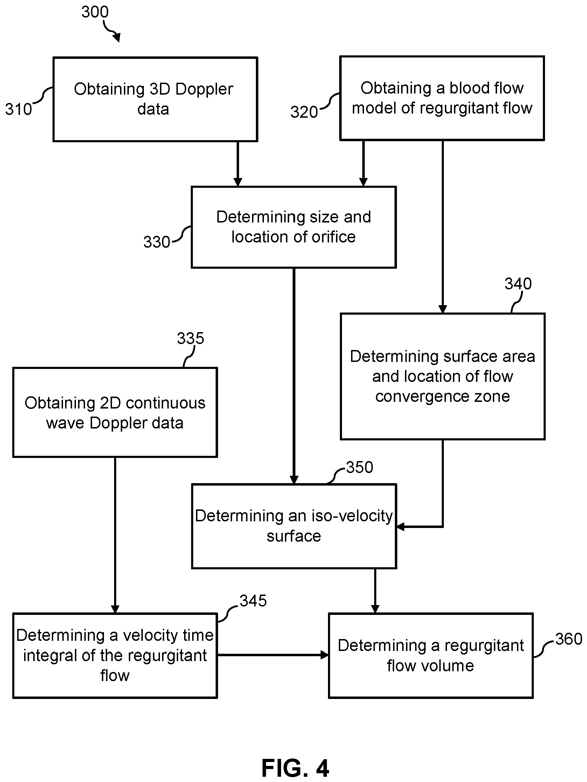

Referring now to Fig. 4, there is depicted a flow diagram of a method 300 for automatically determining an iso-velocity surface for use in 2D PISA measurements according to a proposed embodiment.

In step 310, 3D Doppler data representative of regurgitant flow associated with an orifice in a cardiac valve is obtained, wherein the 3D Doppler data comprises at least two 3D Doppler data frames from a temporal sequence. Each Doppler data frame comprises velocity values. A Doppler data frame can be understood as a single frame of Doppler data, i.e., describing a single moment in time, comprising velocity values. Likewise, each blood flow model frame comprises blood flow velocity values.

In step 320, a blood flow model of the regurgitant flow, wherein the blood flow model comprises a model of the orifice and blood flow velocity values through and around the orifice, is obtained, wherein the blood flow model comprises at least two blood flow model frames from a temporal sequence. A blood flow model frame can be understood as a single frame of a (temporal) blood flow model, i.e., describing a single moment in time, comprising blood flow velocity values.

In this way, a dynamic view of the regurgitant flow over time can be provided, which can capture variations within the cardiac cycle and potentially lead to more accurate assessments.

In step 330, a size and location of the orifice in the blood flow model is determined based on the 3D Doppler data frame comprising the largest regurgitant jet or the largest orifice area respectively. Focusing on the frame with the greatest regurgitation can ensure that the determined iso-velocity surface accurately reflects the extent of the regurgitation. The largest regurgitant jet can be understood as the longest jet, the jet with the largest surface area, or any other suitable metric, as the skilled person would understand.

In step 340, a surface area and location of a flow convergence zone in the blood flow model is determined based on blood flow velocity values from the blood flow model frame comprising the largest regurgitant flow or the largest orifice area respectively. Focusing on the frame with the greatest regurgitation can ensure that the determined iso-velocity surface accurately reflects the extent of the regurgitation.

In step 350, an iso-velocity surface of the blood flow model is determined based on the determined surface area and location of the flow convergence zone (based on blood flow velocity values from the blood flow model frame comprising the largest regurgitant flow or the largest orifice area respectively) and the determined size and location of the orifice (based on the 3D Doppler data frame comprising the largest regurgitant jet or the largest orifice area respectively).

In an alternative embodiment, step 330 can comprise determining a size and location of the orifice for each Doppler data frame; step 340 can comprise determining a surface area and location of the flow convergence zone for each blood flow model frame; and step 350 can thus comprise determining an iso-velocity surface for each pair of corresponding frames. This allows for a frame-by-frame analysis, which can provide a detailed temporal representation of the regurgitant flow, facilitating a more nuanced understanding of the condition.

In step 335, 2D continuous wave Doppler data representative of the regurgitant flow is obtained. In some embodiments, the 2D continuous wave Doppler data can actually be captured/measured such that the method 300 is more self-contained.

In step 345, a velocity time integral of the regurgitant flow is determined based on the obtained 2D continuous wave Doppler data. This allows the velocity time integral to be directly automatically determined in the method 300 rather than obtained from an external source, and thus allows the method to be more self-contained.

In other embodiments, however, step 335 can be skipped, and step 345 can instead comprise obtaining the velocity time integral of the regurgitant flow (for example, from an external memory storage or manually input by a user).

In step 360, a regurgitant flow volume is determined based on the iso-velocity surface of the blood flow model and the velocity time integral. This can facilitate a more comprehensive automatic assessment of the regurgitant flow. The total volume of the regurgitation flow is clinically relevant for patient diagnosis and treatment planning.

It should be noted that, in some embodiments, a regurgitant orifice area can be estimated directly from the obtained 3D Doppler data, using the intersection of the 3D Doppler data with a 3D leaflet surface of the blood flow model to provide an area that the flow is going through. The leaflet surface could be considered 3D, or just a planar surface for simplicity. In either case, the estimated regurgitant orifice area (EROA) combined with a velocity time integral (VTI) can then be used to provide regurgitant volume (RVol).

The present invention leverages blood flow models of regurgitant flows (as documented, for example, in WO 2023/020920 A1), which detect flow orifices and then capture and calculate all flow travelling through (and around) that orifice. Color Doppler information (used in a blood flow model, for example) can be used to compute a flow convergence zone in 3D space (similar to a PISA hemisphere, but in 3D). In other words, a flow convergence zone can be calculated from a blood flow model of regurgitant flow (which uses 3D Color Doppler information, acquired by an ultrasound machine, for example).

Using the orifice (location and size/area) and the flow convergence zone (location and surface area), a PISA measurement can be performed by also using a velocity time integral provided manually or automatically. Both the orifice and the flow convergence zone can be approximated to be circular/spherical in order to maintain compatibility with current 2D PISA calculations.

In a specific example, a user can collect a 3D color data set that encompasses the mitral annulus and regurgitation phenomenon. Preferably, the dataset can have the following acquisition parameters: >20 Hz volume rate; >60 cm/s maximum aliasing velocity; no baseline shift performed prior to acquisition. The user can also collect a continuous wave doppler acquisition to obtain the VTI.

With these two datasets, the user can enter the automatic 2D PISA software. Anatomical landmarks can then be detected and a velocity time integral can be computed before performing orifice and flow convergence zone detection on the frame with the largest regurgitant jet. PISA calculations can then be performed. A user can then be provided with a PISA RVol result, a maximum flow rate, and also shown an overlay on the images where the radius and area measurements were taken from. A user could then manually adjust any of the automatic measurements if they disagree.

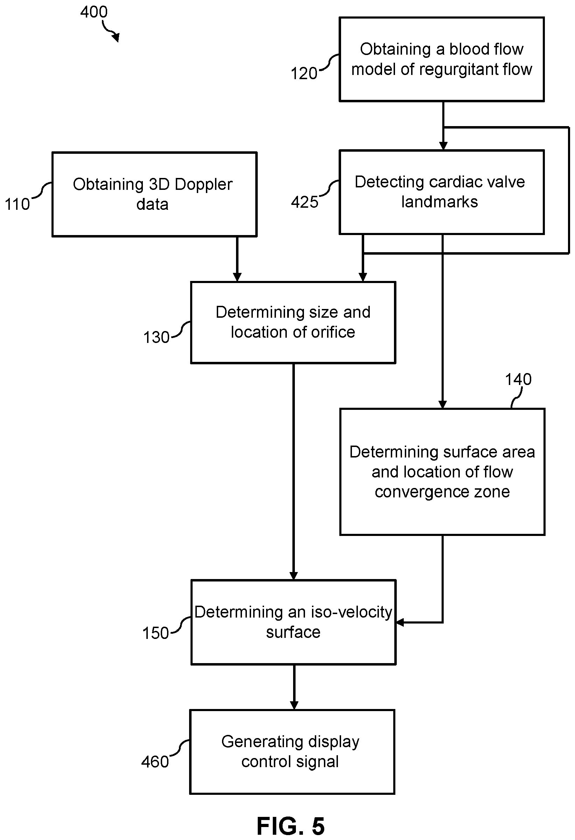

Referring now to Fig. 5, there is depicted a method 400 automatically determining an iso-velocity surface for use in 2D PISA measurements according to a proposed embodiment. Steps 110, 120, 130, 140, and 150 are substantially the same as have been described in relation to method 100 of Fig. 1, and also method 100b of Fig. 3.

Step 425 comprises detecting cardiac valve landmarks in the blood flow model of the regurgitant flow. Determining the size and location of the orifice in step 130 can then further be based on the detected cardiac valve landmarks and/or determining the surface area and location of the flow convergence zone in step 140 can further based on the detected cardiac valve landmarks. Incorporating anatomical landmarks can improve the accuracy of the model by ensuring that the measurements are anatomically consistent.

In step 460, a display control signal is generated, the display control signal describing the determined iso-velocity surface. This facilitates the visualization of the iso-velocity surface, which can aid clinicians in interpreting the data and making informed decisions regarding patient care and/or checking that the automatic determination of the 2D PISA hemisphere agrees with their expectations.

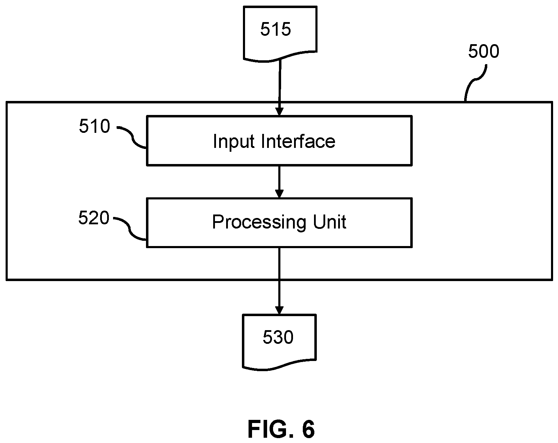

Referring now to Fig. 6, there is depicted a system 500 for automatically determining an iso-velocity surface for use in 2D PISA measurements according to a proposed embodiment. The system 500 comprises an input interface 510 and a processing unit 520.

The input interface 510 is configured to obtain inputs 515. The inputs 515 comprise: 3D Doppler data representative of regurgitant flow associated with an orifice in a cardiac valve; and a blood flow model of the regurgitant flow, wherein the blood flow model comprises a model of the orifice and blood flow velocity values through and around the orifice. The processing unit 520 is configured to determine a size and location of the orifice in the blood flow model based on the 3D Doppler data; determine a surface area and location of a flow convergence zone in the blood flow model based on blood flow velocity values of the blood flow model; and determine an iso-velocity surface of the blood flow model based on the determined surface area and location of the flow convergence zone and the determined size and location of the orifice.

The output 530 of the system 500 can thus comprise the iso-velocity surface of the blood flow model, and in some embodiments, further comprise a display control signal, the display control signal describing the determined iso-velocity surface and/or a determined regurgitant flow volume.

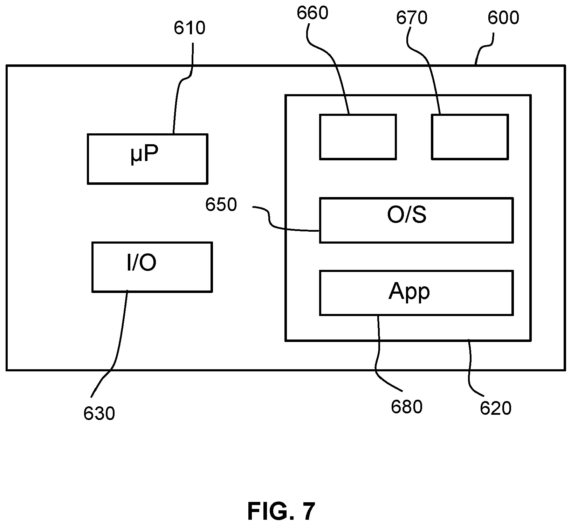

Fig. 7 illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620 and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at start-up, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1 and 3-4, and the system of Fig. 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for automatically determining an iso-velocity surface for use in 2D proximal iso-velocity surface (PISA) measurements, the method comprising:
obtaining a blood flow model of regurgitant flow (120), wherein the blood flow model comprises a model of an orifice in a cardiac valve and blood flow velocity values through and around the orifice;
determining a surface area and location of a flow convergence zone (140) in the blood flow model based on blood flow velocity values of the blood flow model; and
determining an iso-velocity surface (150) of the blood flow model based on the determined surface area and location of the flow convergence zone.

2. The computer-implemented method of claim 1, wherein the method further comprises:
obtaining 3D Doppler data (110) representative of regurgitant flow associated with the orifice in the cardiac valve; and
determining a size and location of the orifice (130) in the blood flow model based on the 3D Doppler data;
wherein determining the iso-velocity surface of the blood flow model is further based on the determined size and location of the orifice.

3. The computer-implemented method of claim 2, wherein the size of the orifice is determined as a diameter of a circle and surface area of the flow convergence zone is determined as spherical.

4. The computer-implemented method of claim 2 or 3, wherein the 3D Doppler data comprises at least two 3D Doppler data frames from a temporal sequence, and wherein the blood flow model comprises at least two blood flow model frames from a temporal sequence.

5. The computer-implemented method of claim 4, wherein the size and location of the orifice is based on the 3D Doppler data frame comprising the largest regurgitant jet or the largest orifice area respectively.

6. The computer-implemented method of claim 4 or 5, wherein the surface area and location of the flow convergence zone is based on the blood flow velocity values from the blood flow model frame comprising the largest regurgitant flow or the largest orifice area respectively.

7. The computer-implemented method of claim 4, wherein a size and location of the orifice is determined for each Doppler data frame (330); a surface area and location of the flow convergence zone is determined for each blood flow model frame (340); and an iso-velocity surface is determined for each pair of corresponding frames (350).

8. The computer-implemented method of any of claims 2 to 7, further comprising detecting cardiac valve landmarks in the blood flow model (425) of the regurgitant flow, and wherein determining the size and location of the orifice is further based on the detected cardiac valve landmarks and/or wherein determining the surface area and location of the flow convergence zone is further based on the detected cardiac valve landmarks.

9. The computer-implemented method of any of claims 1 to 8, wherein the iso-velocity surface comprises a spherical surface.

10. The computer-implemented method of any of claims 1 to 10, wherein the method further comprises: obtaining a velocity time integral of the regurgitant flow; and determining a regurgitant flow volume (360) based on the iso-velocity surface of the blood flow model and the velocity time integral.

11. The computer-implemented method of claim 10, wherein the method further comprises: obtaining 2D continuous wave Doppler data representative of the regurgitant flow (335); and determining the velocity time integral (345) based on the obtained 2D continuous wave Doppler data.

12. A computer program comprising code means for implementing the method of any preceding claim when said program is run on a processing system.

13. A system (500) for automatically determining an iso-velocity surface for use in 2D proximal iso-velocity surface (PISA) measurements, the system comprising:
an input interface (510) configured to:
obtain a blood flow model of the regurgitant flow, wherein the blood flow model comprises a model of an orifice in a cardiac valve and blood flow velocity values through and around the orifice; and
a processing unit (520) configured to:
determine a surface area and location of a flow convergence zone in the blood flow model based on blood flow velocity values of the blood flow model; and
determine an iso-velocity surface of the blood flow model based on the determined surface area and location of the flow convergence zone.

14. The system of claim 13, wherein the input interface (510) is further configured to:
obtain 3D Doppler data representative of regurgitant flow associated with the orifice in the cardiac valve; and
wherein the processing unit (520) is further configured to:
determine a size and location of the orifice in the blood flow model based on the 3D Doppler data; and
wherein determining the iso-velocity surface of the blood flow model is further based on the determined size and location of the orifice.

15. The system of claim 13 or 14, wherein the iso-velocity surface comprises a spherical surface.
